# EUROPÄISCHE PATENTANMELDUNG

(11) **EP 4 321 133 A2**
(43) Veröffentlichungstag der Anmeldung: **14.02.2024**
(21) Anmeldenummer: 23219071.0
(22) Anmeldetag: 16.08.2021
(51) Int. Cl.: A61F 2/38

(54) **TIBIAKOMPONENTE EINER KNIEGELENKENDOPROTHESE MIT TIBIAPLATTE UND VERANKERUNGSKIEL**

(62) Teilanmeldung aus: 21000232.5
(71) Anmelder: Waldemar Link GmbH & Co. KG, 22339 Hamburg (DE)
(72) Erfinder: BAUER, Eckhard, 24159 Kiel (DE); LINK, Helmut D., 22397 Hamburg (DE)
(74) Vertreter: Glawe, Delfs, Moll

(57) **Zusammenfassung**

Tibiakomponente einer Kniegelenkendoprothese umfassend eine sich lateral erstreckende Tibiaplatte (91), einen nach distal abragenden Verankerungskiel (8) mit einem Schaftstück (82) und mindestens einem sich nach lateral erstreckenden flügelartigen Ausleger (83). In einem Implantationszustand sind die Tibiaplatte (91) und der Verankerungskiel (8) mittels lösbarer Befestigungselemente miteinander verbunden. Für einen Explantationszustand ist die Tibiaplatte (91) von dem Verankerungskiel (8) abnehmbar. Erfindungsgemäß ist an der Tibiaplatte (91) mindestens ein proximal entnehmbarer Abstandshalter (7) vorgesehen, dessen Stirnseite (72) einen Anschlag für den Ausleger (83) bildet und dazu ausgebildet ist, zwischen dem Ausleger (83) und der Tibiaplatte (91) einen Freiraum (6) mit einer definierten Mindestweite als Sägeschlitzraum zu schaffen. Mit dem entnehmbaren Abstandshalter kann eine positive Kontrolle über die Abstände auch im Auslegerendbereich erreicht werden. Eine unerwünschte Verengung des Freiraums kann damit wirksam vermieden werden. Um bei einer Explantation den Freiraum hindernisfrei für eine Säge zu machen, ist der Abstandshalter proximal entnehmbar ausgeführt. Ein ungehinderten Freischneiden ist somit frei. Da kein Abstandshalter durchtrennt zu werden braucht, entstehen auch keine unerwünschten Späne aus körperfremdem Material. Die Sicherheit der Explantation wird erhöht und die Gefahr von Infektionen verringert. Die Erfindung betrifft ferner ein Verfahren zur Extraktion sowie ein modulares Tibiakomponenten-System.

## Beschreibung

Die Erfindung betrifft eine Tibiakomponente einer Kniegelenkendoprothese. Die Tibiakomponente umfasst eine sich lateral erstreckende Tibiaplatte, einen nach distal abragenden Verankerungskiel mit einem Schaftstück und mindestens einem sich nach lateral erstreckenden flügelartigen Ausleger.

Kniegelenkendoprothese weisen in der Regel zwei Komponenten auf, eine die am proximalen Ende der Tibia (Tibiakopf) angeordnet ist und eine weitere, die am distalen Ende des Femurs angeordnet ist. Derartige Prothesen sind bekannt und sind zur Anpassung an individuelle Besonderheiten in verschiedenen Grö-βen und Arten verfügbar. Bei der Implantation unterscheidet man zwischen zementierter und nicht zementierter Variante. Um für alle Varianten eine passende Endoprothese bereitstellen zu können, sind Modulsysteme bekannt geworden (z.B. US 7,357,817 B2). Sie ermöglichen es, bspw. für einen in der Tibia zu befestigenden Verankerungskiel verschiedene Tibiaplatten auszuwählen, insbesondere kann zwischen zementierter oder zementfreier Version gewählt werden. Die zementfreie Version bietet den Vorteil, dass durch einwachsendes Knochenmaterial eine hohe Befestigungssicherheit erreicht werden kann, was der Langlebigkeit der Prothese zugute kommt. Dennoch ist die Lebensdauer derartiger Endoprothesen begrenzt, und es kommt zusehends häufiger zu Revisionsoperationen, bei denen die bereits implantierte Prothese und insbesondere deren Tibiakomponente entfernt werden muss. Die Explantation erweist sich in der Praxis häufig als problematische. Eine schonende Explantation, bei der möglichst viel Knochenmaterial erhalten und nur wenig abgetragen zu werden braucht, ist für den Patienten von erheblichem Wert. Bei zweiteiligen Tibiakomponenten tritt das Problem auf, die Tibiaplatte auch dann schonend entfernen zu können, wenn bereits erhebliches Einwachsen von Knochenmaterial stattgefunden hat. Darüber hinaus erweist es sich häufig als besonders schwierig, auch den Verankerungskiel zu entfernen. Häufig kann dies zu erheblichem Knochenverlust führen.

Zur Abhilfe ist es bekannt, die Tibiaplatte mittels einer zentralen Konusverbindung auf den Verankerungskiel aufzusetzen und zwar so, dass zwischen ihnen ein Freiraum in Gestalt eines Sägeschlitzraum verbleibt (EP 1 674 052 B1). Eine weitere Steckverbindung ist an dem freien Ende der Ausleger des Verankerungskiels vorgesehen, als Sicherung gegen ein unerwünschtes Verdrehen der Tibiaplatte gegenüber dem Verankerungskiel. Diese weitere Steckverbindung ist aus einem schneidbarem Material hergestellt, so dass bei einem Freischneiden der Tibiaplatte mittels einer in den Sägeschlitzraum eingeführten Säge diese weitere Steckverbindung ohne weiteres durchtrennt werden kann. Bei nicht exakt paralleler Ausrichtung von Verankerungskiel zur Tibiaplatte, was in der Praxis leicht durch unsymmetrische Belastung verursacht werden kann, verengt sich bedingt durch die erhebliche laterale Erstreckung der Tibiaplatte der Freiraum zu einem der äußeren Enden hin. In der Folge reicht der Freiraum dort nicht mehr aus für das Sägeblatt, was das Durchtrennen des Knochenmaterials erschwert.

Der Erfindung liegt die Aufgabe zugrunde, eine verbesserte Tibiakomponente zu schaffen, die diese Nachteile vermeidet.

Die erfindungsgemäße Lösung liegt in den Merkmalen der unabhängigen Ansprüche. Vorteilhafte Weiterbildungen sind Gegenstand der abhängigen Ansprüche.

Bei einer Tibiakomponente einer Kniegelenkendoprothese umfassend eine sich lateral erstreckende Tibiaplatte, einen nach distal abragenden Verankerungskiel mit einem Schaftstück und mindestens einem sich nach lateral erstreckenden flügelartigen Ausleger, wobei in einem Implantationszustand die Tibiaplatte und der Verankerungskiel mittels lösbarer Befestigungselemente miteinander verbunden sind, und wobei für einen Explantationszustand die Tibiaplatte von dem Verankerungskiel abnehmbar ist, ist erfindungsgemäß an der Tibiaplatte mindestens ein proximal entnehmbarer Abstandshalter vorgesehen ist, dessen Stirnseite einen Anschlag für den Ausleger bildet und der dazu ausgebildet ist, zwischen dem Ausleger und der Tibiaplatte einen vorzugsweise schlitzartigen Freiraum mit einer definierten Mindestweite zu schaffen.

Unter einem Verankerungskiel (Verankerungsschaft) einer Endoprothese wird eine Struktur verstanden, die zur Verankerung der Endoprothese in einem Knochen, insbesondere einem Markraum der Tibia, vorgesehen ist und einen Schaft sowie seitlich daran angeordnete flügelartige Ausleger umfasst.

Unter einem Implantationszustand wird ein Montagezustand der Tibiakomponente verstanden, den sie im implantierten Zustand aufweist. Häufig ist dies auch der Zustand, mit dem die Implantation durchgeführt wird. Das gilt insbesondere im Fall einer Vormontage, beispielsweise bei bereits miteinander verbundenen Verankerungskiel und Tibiaplatte.

Unter einem Explantationszustand wird ein Zustand verstanden, der sich von dem Implantationszustand unterscheidet und zur Explantation realisiert ist.

Der schlitzartige Freiraum ist vorzugsweise bezüglich seiner Weite so bemessen, dass sie für den Durchgang eines Sägeblatts oder eines Sägebands ausreicht, um im Freiraum befindliches (Knochen-)Material zu durchtrennen. Er kann so einen Sägeschlitzraum bilden.

Unter "proximal entnehmbar" wird verstanden, dass der Abstandshalter von der proximalen Seite aus dem Freiraum entnommen werden kann, ohne dafür Zugriff auf die distale Seite zu benötigten . (Die distale Seite ist typischerweise fest mit dem Knochen verbunden mittels Knochenzement oder durch eingewachsenes Knochenmaterial und daher nur schwer zugänglich.)

Begriffe wie "distal" und "proximal" sind anatomische Fachbegriffe für Richtungsbezeichnungen. Sie stehen für eine Richtung von der Körpermitte weg bzw. für eine Richtung zur Körpermitte hin. Der Begriff "lateral" bedeutet eine seitwärtsweisende Richtung hin zu einer linken oder rechten Körperseite.

Die Erfindung beruht auf der Erkenntnis, dass dank der entnehmbaren Abstandshalter eine positive Kontrolle über die Abstände auch im Bereich des jeweiligen Endes des Auslegers erreicht werden kann. Der Gefahr einer Verengung des Freiraums durch häufig unvermeidliche Schiefstellung infolge unsymmetrischer Belastung kann damit auf einfache, aber wirksame Weise begegnet werden. Es ergibt sich jedoch die Schwierigkeit, dass ein hinreichend robuster Abstandshalter typischerweise aus einem solchen Material (wie Titan) besteht, dass nur schlecht von einer Säge für Knochenmaterial geschnitten werden kann. Insoweit besteht ein Zielkonflikt. Die Erfindung löst diesen Zielkonflikt, indem sie den Abstandshalter von proximal entnehmbar ausführt. Auf diese Weise kann vor dem Einsatz der Säge der Abstandshalter entfernt und damit der schlitzartige Freiraum als Sägeschlitz hindernisfrei gemacht werden. Einem ungehinderten Freischneiden steht somit nichts mehr im Wege.

Da somit nur Knochenmaterial durchtrennt zu werden braucht und keine anderen Materialien (nämlich die des Abstandshalters), gelangen auch keine unerwünschten Späne aus körperfremdem Material an oder in den Knochen. Dies ist ein weiterer erheblicher Vorteil, insbesondere in Bezug auf die Verringerung der Infektionsgefahr für den Patienten.

Vorzugsweise weist die Befestigungsöffnung eine für den Durchgang des Abstandshalters ausreichende Weite auf, wobei weiter vorzugsweise die Befestigungsöffnung gestuft ausgeführt ist mit vergrößerter Weite nach proximal. Damit ist das definierte Positionieren und Entnehmen des Abstandshalters vereinfacht, insbesondere wenn die gestufte Befestigungsöffnung eine Schulter als Anschlag für den Abstandshalter aufweist.

Zweckmäßigerweise ist der Abstandshalter im Implantationszustand in eine Befestigungsöffnung der Tibiaplatte eingeschraubt. Damit kann er auf einfache Weise zur Implantation eingesetzt und durch Herausschrauben leicht für die Explantation entnommen werden. Ferner bietet dies die optionale Möglichkeit, gewünschtenfalls eine Feineinstellung des Abstands vorzunehmen, indem der Abstandshalter unterschiedlich weit eingeschraubt wird.

Mit Vorteil weist der Abstandshalter ein Außengewinde auf, das in ein Innengewinde der Befestigungsöffnung eingreift. Auf diese Weise kann eine rationelle Befestigung des Abstandhalters in der Befestigungsöffnung erfolgen. Außerdem wird mit dem Außengewinde der zur Verfügung stehende Bauraum optimal genutzt.

Vorzugsweise ist der Abstandshalter so ausgeführt, dass er eine Doppelfunktion hat und zusätzlich als Halterung für eine von proximal zu betätigende Befestigungsschraube fungiert, die den Verankerungskiel mit der Tibiaplatte verbindet. Damit kann auf raumsparende Weise nicht nur eine definierte Abstandseinstellung, sondern auch eine Befestigung der Tibiaplatte am Ausleger erfolgen. Damit kann der Ausleger seine stützende und aussteifende Wirkung für die Tibiaplatte voll entfalten.

Die Befestigungsschraube ist zweckmäßigerweise koaxial im Abstandshalter angeordnet, wobei sie vorzugsweise im montierten Zustand einen Zugang zu einem Betätigungsorgan des Abstandshalters verschließt. Mit der koaxialen Anordnung wird zum einen eine besonders raumsparende Konstruktion erreicht, was bei gegebenem Bauraum größere Befestigungsschrauben und damit eine robustere Verbindung ermöglicht. Zum anderen bietet die koaxiale Anordnung den Vorteil, dass der Schraubenkopf der Befestigungsschraube die Oberseite des Abstandshalters nahezu vollständig bedecken kann. Zum einen bleibt die Oberseite damit frei von Beeinträchtigungen durch Fremdmaterial, und zum anderen ist bei eingesetzter Befestigungsschraube ein Betätigen, und sei es auch nur unabsichtlich, des Abstandshalters somit mangels Zugänglichkeit blockiert.

Es hat sich bewährt, wenn dem Ausleger gegenüberliegend ein zweiter Ausleger an dem Verankerungskiel vorgesehen ist, der in entsprechender Weise mit einem zweiten Abstandshalter sowie zweiter Befestigungsschraube an der Tibiaplatte zusammenwirkt. Typischerweise ist eine symmetrische Anordnung der Ausleger vorgesehen, um so eine beidseitige symmetrische Unterstützung der Tibiaplatte zu erreichen. Zwingend ist eine symmetrische Anordnung jedoch nicht, es kann auch vorgesehen sein, dass Länge und/oder Winkelstellung sich unterscheiden. Vorzugsweise sind die Ausleger so angeordnet, dass sie zwischen sich einen Winkel zwischen 100° und 170° bilden. Damit wird dank des gro-βen Winkels eine breite Abstützung erreicht, die jedoch bei einem von 180° abweichenden Winkel nicht in einer Linie mit dem Schaftstück ist, sondern vorzugsweise ragen die Ausleger nach posterior. Damit kann zusätzlich auch eine Abstützung nach anterior oder posterior (anatomische Fachbegriffe für in Körperrichtung gesehen nach vorne oder nach hinten) bewirkt werden, und in der Folge eine noch bessere Unterstützungswirkung durch den Verankerungskiel erreicht werden.

Es ist zweckmäßig, wenn der Verankerungskiel mit den Auslegern eine plane Oberseite 85 aufweist. Auf diese Weise kann im Zusammenspiel mit der typischerweise planen distalen Unterseite der Tibiaplatte und dem durch die Abstandshalter definierten Abstand eine gleichmäßige Breite des Sägeschlitzraums über die gesamte Erstreckung der Ausleger erreicht werden.

Mit Vorteil ist an der Tibiaplatte eine Öffnung für eine Zentralschraube vorgesehen, mit der der Verankerungskiel an der Tibiaplatte befestigt ist. Mit der Zentralschraube kann eine kräftige lastübertragende Verbindung zwischen Tibiaplatte und Verankerungskiel bereitgestellt werden. Weiter ermöglicht sie es, dass der Verankerungskiel gegen die Wirkung der Abstandshalter zur Tibiaplatte hin gezogen werden kann. Es entsteht so eine Art verspannte Befestigung, die einen zusätzlichen Schutz insbesondere gegen unerwünschte Lockerung bietet.

Mit Vorteil ist die distale Seite der Tibiaplatte mit einer porösen Struktur versehen. Damit werden an der großen Kontaktfläche, mit der die distale Seite auf dem Knochen flächig anliegt, günstige Bedingungen für Knocheneinwuchs und somit gute Befestigung der Tibiaplatte geschaffen. Vorzugsweise ist die poröse Struktur so ausgeführt, dass sie in der Tiefe des Materials miteinander verbundene Poren umfasst. Die Porosität geht somit über eine oberflächliche Porosität hinaus und reicht bis in die Tiefe des Materials. Es werden so Hohlräume geschaffen, die miteinander in Verbindung stehen, wodurch besonders günstige Bedingungen für den Einwuchs von Knochenmaterial geschaffen sind. Mit Vorteil sind die Poren so dimensioniert, dass sie eine Weite von 0,1 bis 1,5 mm, insbesondere zwischen 0,7 und 1,3 mm, aufweisen. Damit ergibt sich ein besonders günstiges Einwuchsverhalten. Zweckmäßig ist, wenn die poröse Struktur in der Tiefe mindestens eine und bis zu drei Schichten von Poren umfasst, was typischerweise eine bevorzugten Dicke der porösen Struktur von etwa 0,6 mm bis 2,5 mm bedeutet.

Zweckmäßigerweise ist vorgesehen, dass die poröse Struktur flächig die distale Seite der Tibiaplatte bedeckt. Damit kann ein solides Einwachsen von Knochenmaterial über die Fläche der distalen Seite der Tibiaplatte erreicht werden, womit sich eine optimale Ankopplung der Tibiaplatte an den resezierten Tibiakopf ergibt.

Im Hinblick auf die erheblichen Belastungen des Kniegelenks, die über die Kondylen der Femurkomponente auf die Tibiaplatte geleitet werden, sind zweckmäßigerweise Verstärkungsrippen an der distalen Seite der Tibiaplatte vorgesehen. Damit kann eine erhebliche Aussteifung der vorzugsweise dünn ausgeführten Tibiaplatte erreicht werden. Damit kann auch dem Effekt entgegengewirkt werden, dass infolge der porösen Struktur bei gleicher Außendicke die Materialstärke des eigentlich tragenden, massiven Teils der Tibiaplatte und damit auch deren Lastaufnahmefähigkeit verringert ist.

Vorzugsweise erstrecken sich die Verstärkungsrippen an der distalen Seite der Tibiaplatte radial von einem Mittelbereich aus zum Rand der Tibiaplatte. Damit erfolgt auf effiziente Weise eine Verstärkung entlang den Hauptbelastungslinien der Tibiaplatte. Hierbei können die Verstärkungsrippen so kräftig ausgeführt sein, dass sie hervorstehen und insbesondere aus der porösen Struktur herausragen. Es kann aber auch vorgesehen sein, dass die Verstärkungsrippen in der porösen Struktur versenkt angeordnet sind. Dies hat den Vorteil, dass an der Oberfläche eine durchgängige poröse Struktur besteht, was ein flächiges Einwachsen von Knochenmaterial begünstigt.

Mit Vorteil ist an der distalen Seite der Tibiaplatte ein umlaufender Rand für die poröse Struktur vorgesehen. Mit dem Rand wird eine eindeutige Begrenzung der porösen Struktur erreicht. Ein weiterer Vorteil des Rands liegt aber darin, dass somit insbesondere an den äußeren Bereichen der porösen Struktur ein unerwünschtes Ausbrechen des mechanisch empfindlicheren porösen Materials aus der porösen Struktur vermieden werden kann.

Zweckmäßigerweise ist eine Breite des Rands nur so groß bemessen, dass die poröse Struktur mindestens die Hälfte, vorzugsweise mehr als 60%, der Fläche der distalen Seite der Tibiaplatte einnimmt. Damit wird vermieden, dass durch einen überbreiten Rand ein zu großer Teil der distalen Unterseite der Tibiaplatte für die poröse Struktur nicht zur Verfügung steht. Insbesondere bei kleinen Größenstufen der Tibiaplatte kann dies sonst ein Problem werden in Bezug auf eine hinreichend sichere Befestigung der Tibiaplatte durch einwachsendes Knochenmaterial. Mit der gewählten Untergrenze ist sichergestellt, dass ausreichend Fläche für den Einwuchs von Knochenmaterial zur Verfügung steht.

Die Erfindung umfasst weiter ein modulares Tibiakomponenten-System von Kniegelenkendoprothesen, umfassend eine oder mehrere der vorstehend beschriebenen Tibiakomponenten, wobei mindestens zwei verschiedene Tibiaplatten vorgesehen sind, die wahlweise mit einem Verankerungskiel verbindbar sind, wobei sich die Tibiaplatten hinsichtlich ihrer Ausgestaltung, insbesondere hinsichtlich ihrer knochenkontaktierenden Oberflächen, unterscheiden. Mit einem solchen modularen System können verschiedene Komponenten kombiniert werden, beispielsweise verschiedene Tibiaplatten mit einem Verankerungskiel, und/oder es wird aus einer Auswahl von mehreren Verankerungskielen ein geeigneter Verankerungskiel ausgewählt und mit einer der Tibiaplatte kombiniert. Vorzugsweise umfasst das Tibiakomponenten-System verschiedenen Tibiaplatten in unterschiedlichen Größenstufen und/oder mindestens zwei verschieden Verankerungskiele in unterschiedlichen Größenstufen. Insgesamt können durch solche Kombinationen eine weitgehende Anpassung an die individuellen Anforderungen jedes Patienten erreicht werden bei noch überschaubarer Anzahl von verschiedenen Komponenten.

Im Hinblick auf eine solide Befestigung über alle Größen hinweg ist es zweckmäßig, dass bei Tibiaplatten in verschiedenen Größenstufen die Breite des Randes bei kleineren Größenstufen geringer ist. Durch die schmalere Ausführung des Rands wird bei den kleineren Größenstufen somit mehr Fläche bereitgestellt, die für eine poröse Struktur genutzt werden kann. Auf diese Weise wird auch bei den kleineren Größenstufen eine ausreichend sichere Befestigung der distalen Seite der Tibiaplatte am Knochenkopf durch einwachsendes Knochenmaterial sichergestellt. Hierbei werden unter Größenstufen relative qualitative Angaben in Bezug auf die Abmessungen der einzelnen Komponenten verstanden, beispielsweise "S" für eine kleine Größe, "M" für eine mittlere Größe, "X" für eine große und "XL" für eine übergroße Ausführung. Es versteht sich, dass auch mehr als vier verschiedene Größenstufen vorgesehen sein können.

Die Erfindung bezieht sich ferner auf ein Instrumentarium für die Tibiakomponente, das als Extraktionswerkzeuge umfasst eine Säge mit einem Sägeblatt oder Sägeband, dessen Sägebreite höchstens so groß ist wie die Mindestweite des Freiraums, sowie eine Ausstech-Hülse, deren Innenkontur zur Aufnahme der lateralen Außenkontur des Verankerungskiel mit seinen Auslegern ausgebildet ist, und ein Kopplungselement, welches zugfest mit dem Schaftteil des Verankerungskiels verbindbar ist. Unter der "lateralen Außenkontur" wird die maximale Außenkontur in einer Ebene senkrecht zur proximal-distalen Richtung verstanden. Damit wird eine lateral umlaufende Umhüllende beschrieben.

Dank der Extraktionswerkzeuge kann eine patientenschonende Explantation erreicht werden. Durch die Säge mit einem Sägeblatt oder Sägeband der angegebenen Breite ist sichergestellt, dass diese in den Sägeschlitz eindringen kann und das gewünschte Durchtrennen von Knochenmaterial an der distalen Seite der Tibiaplatte bewirken kann, um so die Tibiaplatte freizuschneiden und abnehmen zu können. Mittels der Ausstech-Hülse kann in einem nachfolgenden Schritt die unmittelbare Umgebung des Verankerungskiels von proximal her abgetrennt werden von dem eigentlichen Knochenmaterial des Tibiakopfs bzw. der Tibia insgesamt. Durch Einbringen der Ausstech-Hülse kann der Verankerungskiel insbesondere in seinem ausladenden proximalen Bereich von dem umliegenden Knochenmaterial freigeschnittenen werden. Das zugfest mit dem Schaftteil des Verankerungskiels verbindbare Kopplungselement bietet den Vorteil, dass dieses einen Ansatzpunkt bietet um, insbesondere nach dem Freischneiden, den noch in der Tibia befindlichen Teil des Verankerungskiels herausziehen zu können. Das Instrumentarium erleichtert so das Entfernen des Verankerungskiels, und zwar unter gleichzeitiger Schonung des Knochenende des durch ein definiertes Freischneiden des Verankerungskiels dank der erfindungsgemäßen Ausstech-Hülse.

Ferner erstreckt sich die Erfindung auf ein Verfahren zur Extraktion der Tibiakomponente, wobei nach Freilegen der Tibiaplatte vorgesehen ist, dass die Befestigungsschrauben aus der Tibiaplatte gelöst und entfernt werden, die Abstandshalter von der Tibiaplatte entfernt werden, mittels einer Säge mit einem Sägeblatt oder Sägeband, dessen Sägebreite höchstens so groß ist wie die Mindestweite des Freiraums, entlang der distalen Seite der Tibiaplatte eingewachsenes Knochenmaterial durchtrennt wird, und die Tibiaplatte abgenommen wird. Dies umfasst insbesondere mit Vorteil die Schritte Aufsetzen einer Ausstech-Hülse, deren Innenkontur zur Aufnahme der lateralen Außenkontur des Verankerungskiel mit seinen Auslegern ausgebildet ist, auf eine durch das Entfernen der Tibiaplatte freigelegte Resektionsebene, und Freischneiden des Verankerungskiels durch Bewegen der Ausstech-Hülse nach distal, vorzugsweise mittels einer Spanneinrichtung, die an einem proximal an dem Schaftstück befestigten Koppelelement angeordnet ist.

Weiter umfasst das Verfahren optional ein Ankoppeln eines Abziehinstruments an das Schaftstück und/oder das Koppelelement, Extrahieren des freigeschnittenen Verankerungskiels durch Aufbringen einer Zugkraft, vorzugsweise mittels eines Gleithammers.

Mit dem Gleithammer kann auf schonende Weise eine ausreichende Betätigungskraft zum Herausziehen des freigeschnittenen Verankerungskiels aus dem Tibiakopf aufgebracht werden. So kann insgesamt mit verhältnismäßig wenig Knochenverlust die Tibiakomponente explantiert werden.

Zur näheren Erläuterung wird auf vorstehende Beschreibung zu der Tibiakomponente und den Vorgängen bei der Explantation verwiesen.
- Fig. 1: eine Frontalansicht eines Ausführungsbeispiels der Tibiakomponente einer Kniegelenkendoprothese;
- Fig. 2A, B: Explosionsansichten zu Fig. 1 mit getrennter bzw. verbundener Tibiaplatte und Verankerungskiel;
- Fig. 3: eine perspektivische Explosionsansicht zu Fig. 2A von schräg distal gesehen;
- Fig. 4: eine Lateralansicht zu Figur 1;
- Fig. 5: eine perspektivische Ansicht darstellend teilmontierte Abstandshalter und Befestigungsschrauben;
- Fig. 6: eine perspektivische Ansicht mit der Tibiakomponente und einer oszillierenden Säge am Freiraum;
- Fig. 7: eine perspektivische Ansicht mit der Tibiakomponente und einer Bandsäge am Freiraum;
- Fig. 8A-C: perspektivische distale Schrägansichten der Tibiaplatte mit poröser Struktur in drei Varianten;
- Fig. 9: eine Aufsicht von distal zu Figur 8A;
- Fig. 10: eine vergrößerte Schnittansicht zu Figur 9;
- Fig. 11: Darstellungen zu Tibiakomponenten in verschiedenen Größen eines modularen Tibiakomponenten-Systems; und
- Fig. 12A-I: Ansichten zu Instrumenten und Verfahrensschritten für eine Explantation.

Nachfolgend wird die Erfindung erläutert anhand eines Beispiels für eine Tibiakomponente einer Kniegelenkendoprothese. Sie ist dazu ausgebildet, am proximalen Knochenende (Knochenkopf) einer Tibia implantiert zu werden. Die in ihrer Gesamtheit mit der Bezugsziffer 9 bezeichnete Tibiakomponente besteht vorzugsweise aus einer Titanlegierung. Sie kann aber auch aus einem anderen biokompatiblen Material bestehen, beispielsweise Kobalt-Chrom-Molybdän (CoCrMo).

Die Kniegelenkendoprothese umfasst eine am Femurknochen (nicht dargestellt) anzuordnende femorale Komponente (nicht dargestellt) sowie die am proximalen Ende eines Tibiaknochens 99 anzuordnen Tibiakomponente 9. Diese umfasst eine Tibiaplatte 91, die lateral ausgedehnt auf einem resezierten Knochenkopf der Tibia angeordnet ist. Auf der proximalen Seite (Oberseite) der Tibiaplatte 91 ist eine Aufnahme 90 vorgesehen, in welche ein Lagerstück (nicht dargestellt) der Kniegelenkendoprothese anzuordnen ist. Die gegenüberliegende distale Seite 92 der Tibiaplatte 91 ist ausgebildet zur Anlage an der Oberfläche des Knochenkopfs der Tibia 99, und ist dazu mit einer porösen Struktur zum Einwuchs von Knochenmaterial versehen.

Zur Befestigung der Tibiaplatte 91 ist ein Verankerungskiel 8 vorgesehen, welcher nach distal abragt. Er weist ein Schaftstück 82 und ein sich daran distal anschließendes Konusstück 81 auf. Das Konusstück 81 ist dazu ausgebildet, ggf. einen in den Markkanal der Tibia ragenden Steckschaft aufzunehmen. Nach lateral schließen sich an das Schaftstück 82 jeweils ein flügelartiger Ausleger 83 sowohl zur linken wie zur rechten Seite an, an deren freien Ende eine Spannhülse 84 angeordnet ist. Die flügelartigen Ausleger 83 fungieren als Tragarme für die Tibiaplatte 91. Die Tibiaplatte 91 ist somit an drei Punkten mit dem Verankerungskiel 8 verbunden, nämlich zentral an dem Schaftstück 82 und jeweils links- und rechtsseitig mit den an freien Enden der flügelartigen Ausleger 83 angeordneten Spannhülsen 84. Dazu sind an der Tibiaplatte 91 zwei Verschraubungslöcher als Befestigungsöffnungen 94 links und rechts vorgesehen, die mit der jeweiligen Spannhülse 84 fluchten.

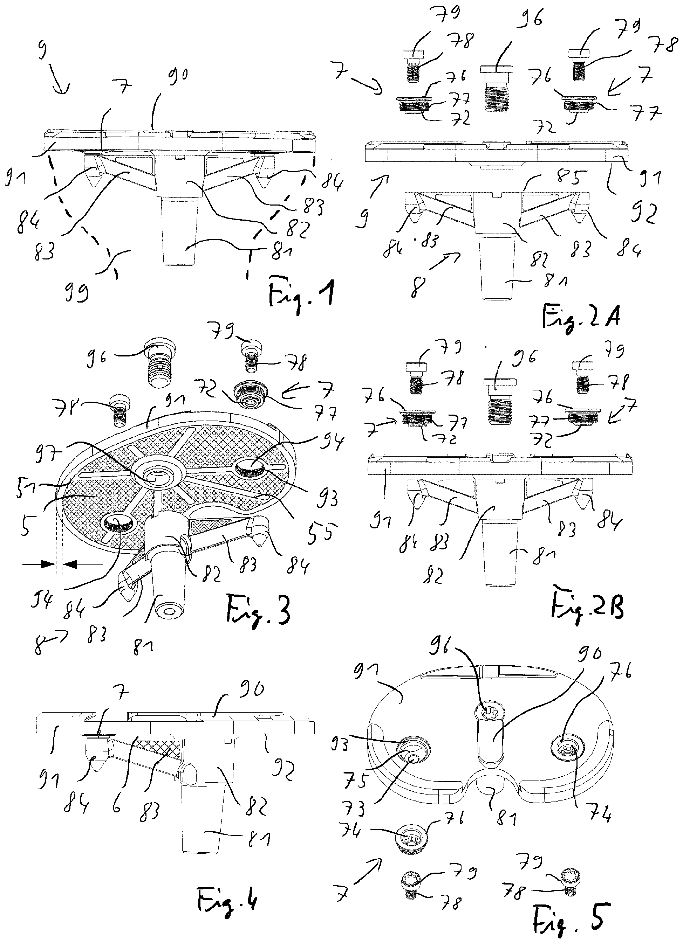

Hierbei liegen die flügelartigen Fortsetzung 83 nicht bündig an der Unterseite 92 der Tibiaplatte 91 an, sondern sind mit einem definierten Abstand gehaltert. In die Befestigungsöffnungen 94 sind Abstandshalter 7 mit einem Außengewinde 77 eingeschraubt (s. Fig. 1 und 4), welche einen definierten Abstand zu den Spannhülsen 84 herstellen.

Dies wird nachfolgend näher unter Bezugnahme auf die Figuren 2A, B sowie Figur 3 erläutert. In der Tibiaplatte 91 sind drei Öffnungen vorgesehen, welche zur Verbindung der Tibiaplatte 91 mit dem Verankerungskiel 8 und dessen flügelartigen Ausleger 83 dienen. In der Mitte ist eine zentrale Öffnung 97 vorgesehen, welche zur Aufnahme einer verhältnismäßig groß dimensionierte Zentralschraube 96 ausgebildet ist. Diese dient dazu, eine Verbindung zu dem Verankerungskiel 8 zu schaffen, über welche die über das Kniegelenk der Kniegelenkendoprothese auf die Tibiaplatte 91 wirkende Hauptbelastung in den Verankerungskiel 8 geleitet wird. Zur Verstärkung und der Schaffung einer Auflagefläche kann die zentrale Öffnung 97 an ihrer Mündung mit einem Bund versehen sein. Zu den lateralen Rändern der Tibiaplatte 91 hin versetzt sind weiter die Befestigungsöffnungen 94 vorgesehen. Diese weisen ein Innengewinde 93 auf und sind so positioniert, dass sie mit den Spannhülsen 84 am Ende der flügelartigen Ausleger 83 fluchten. Vorzugsweise sind die Befestigungsöffnungen 94 jeweils als Stufenbohrung vorgesehen, deren proximaler Bereich eine größere Weite aufweist.

Die Abstandshalter 7 sind mit ihren Außengewinde 77 dazu ausgebildet, in die Innengewinde 93 der Befestigungsöffnungen 94 eingeschraubt zu werden. Sie sind im Implantationszustand der Tibiakomponente 9 so weit eingeschraubt, dass sie mit ihrer distalen Stirnseite 72 aus der distalen Unterseite 92 der Tibiaplatte 91 herausstehen. Somit liegen im Implantationszustand die Stirnseiten 72 der Abstandshalter 7 auf der proximalen Seite der Spannhülsen 84 auf. Auf diese Weise ist durch die herausstehende Stirnseite 72 der Abstandshalter 7 ein Maß für den Abstand eingestellt. Damit die genaue Einstellung des Abstands nicht jedes Mal von Hand erfolgen muss, ist zweckmäßigerweise an den Abstandshaltern 7 im proximalen Bereich ein seitlich vorstehender Umlaufrand 76 ausgeformt. Die Dicke dieses Umlaufrand 76 und die Länge des Abstandseinsatzes 7 sind so aufeinander abgestimmt, dass die Stirnseite 72 um das gewünschte Abstandsmaß aus der distalen Unterseite 92 der Tibiaplatte 91 herausragt, wenn der Umlaufrand 76 auf der der Tibiaplatte 91 zum Anlage kommt. Diese Anlage kann auf der proximalen Oberseite der Tibiaplatte 91 erfolgen; bevorzugt ist aber, wenn der Abstandshalter 7 in die jeweilige Befestigungsöffnung 94 versenkt ist und der Umlaufrand 76 dabei auf einer Schulter 75 der Stufenbohrung aufliegt. Auf diese Weise braucht der Operateur sich nicht um die Einstellung des Abstandsmaßes zu kümmern, sondern schraubt den Abstandshalter 7 soweit ein, bis der Umlaufrand 76 an der Schulter 75 in der jeweiligen Befestigungsöffnung 94 anschlägt. Damit ist automatisch das richtige Maß für das Herausstehen der Stirnseite 72 eingestellt.

Wird dann die Tibiaplatte 91 auf dem Verankerungskiel 8 in an sich bekannter Weise montiert und die Zentralschraube 96 angezogen, kommen die Spannhülsen 84 zur Anlage an der herausstehenden Stirnfläche 72 der Abstandshalter 7, wodurch dann zwangsläufig der definierte Abstand eingestellt und der gewünschte Freiraum 6 mit definierten Abstand bis hin zu den äu-βeren freien Enden der flügelartigen Ausleger 83 geschaffen ist.

Zur besseren Fixierung sind zusätzlich noch Befestigungsschrauben 78 für die Abstandshalter 7 vorgesehen. Dazu ist der Abstandshalter 7 als Hülse ausgeführt mit einer koaxialen Innenöffnung 73, durch welche die Befestigungsschraube 78 durchgesteckt ist. Damit kann die Befestigungsschraube 78 mit ihrem Gewinde in das entsprechende Gegengewinde der Spannhülse 84 eingreifen, und durch Festziehen der Befestigungsschraube 78 wird die Spannhülse 84 gegen den Abstandshalter 7 mit dem definierten Abstand gespannt. Damit ist der Verankerungskiel 8 zusätzlich an der Tibiaplatte 91 befestigt und gesichert.

Auf diese Weise wird ein definierter Abstand zwischen der Unterseite 92 der Tibiaplatte 91 und den flügelartigen Auslegern 83 des Verankerungskiels 8 eingestellt. Mit dem definierten Abstand wird der schlitzartige Freiraum 6 zwischen Tibiaplatte und 91 und dem Verankerungskiel 8 mit seinen flügelartigen Auslegern 83 geschaffen, der als Sägeschlitz fungiert. Dies ist in den Figuren 1 und 4 dargestellt, und der als Sägeschlitz fungierende Freiraum 6 ist durch die mit gestrichelter Linie dargestellten Ellipsen hervorgehoben.

Mit der auf diese Weise erfolgten Vormontage des Verankerungskiels 8 an die Tibiaplatte 91 ist die Tibiakomponente 9 im Implantationszustand.

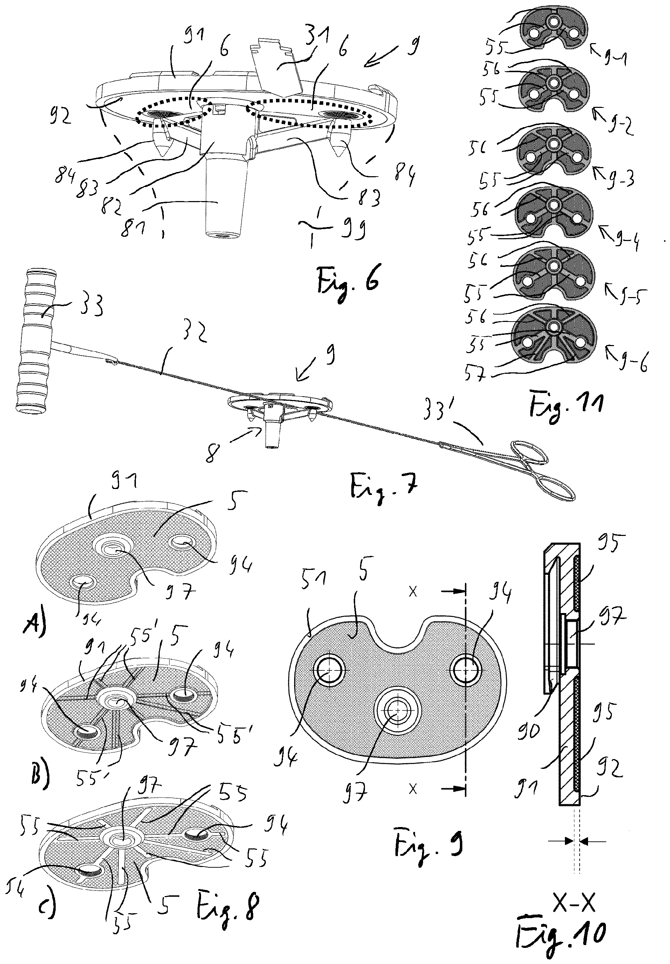

Für den Explantationszustand sind die Abstandshalter 7 zu entfernen. Dazu werden zuerst die Befestigungsschrauben 78 gelöst und entnommen. Dadurch wird an der Oberseite der Abstandshalter 7 eine von dem Kopf 79 der Befestigungsschraube 78 verdeckte Schraubenzieheraufnahme 74 zugänglich. Damit ist es ermöglicht, im nächsten Schritt die Abstandshalter 7 aus den Befestigungsöffnungen 94 zu entfernen. Dieser Zustand ist in Figur 6 dargestellt. Man erkennt, dass nunmehr der Freiraum 6 zwischen der Oberseite 85 der flügelartigen Ausleger 83 des Verankerungskiels 8 und der Unterseite 93 der Tibiaplatte 91 von der Seite her zugänglich ist für ein Sägeblatt 31. Mit dem Sägeblatt 31 einer oszillierenden Knochensäge kann somit in den Freiraum 6 eingewachsenes Knochenmaterial durchtrennt werden und die Tibiaplatte 91 somit freigeschnitten werden.

Eine Alternative hierzu ist Figur 7 dargestellt. Es wird statt einer Säge mit Sägeblatt 31 eine Bandsäge 32 verwendet. Sie ist an ihren beiden Enden mit jeweils einem Zuginstrument 33, 33` versehen. Durch Hin- und Herbewegen der Zuginstrumente 33, 33` kann das Sägeband 32 das im Freiraum 6 eingewachsene Knochenmaterial durchtrennen und so die Tibiaplatte 91 freischneiden.

Die Tibiaplatte 91 ist an ihrer distalen Seite 92 mit einer porösen Struktur 5 versehen (s. Figur 9). Dazu ist eine (oder mehrere) großflächige taschenartige Ausnehmung 95 an der Unterseite ausgebildet, in der die poröse Struktur 5 angeordnet ist. Die poröse Struktur 5 ist so ausgeführt, dass sie in der Tiefe des Materials (der porösen Struktur) verbundene Poren umfasst. Es werden so Hohlräume geschaffen, die miteinander in Verbindung stehen wodurch sich günstige Bedingungen für den Einwuchs von Knochenmaterial ergeben. Dazu sind die Poren vorzugsweise im Bereich von 0,4 bis 1 mm dimensioniert. Die Tiefe der Ausnehmung 95 beträgt etwa 1 bis 2,5 mm (siehe Figur 10). Damit kann die poröse Struktur 5 mindestens eine und bis zu drei Schichten von in der Tiefe miteinander verbundenen Poren umfassen.

Die großflächige taschenartige Ausnehmung 95 ist umgeben von einem umlaufenden Rand 51 (siehe Figur 3). Er weist eine Breite von etwa 1,0 bis 2,5 mm auf. Im Übrigen ist die distale Seite 92 der Tibiaplatte 91 nahezu vollflächig (bis auf die Löcher 94, 97 für die Befestigung) mit der porösen Struktur 5 versehen, wie insbesondere in Figur 8A dargestellt ist. In Figur 8B und 8C sind Varianten dargestellt, bei denen Verstärkungsrippen 55 vorgesehen sind, die ausgehend von der zentralen Öffnung 95 sich radial hin zum Rand 51 erstrecken. Bei der in Figur 8C dargestellten Variante erreichen die Verstärkungsrippen den Rand 51 jedoch nicht, sondern am Rand 51 sind poröse Verbindungsflächen 54 für die poröse Struktur 5 zu jeder Seite der jeweiligen Verstärkungsrippen 55 vorgesehen. Bei der in Figur 8B dargestellten Variante sind die Verstärkungsrippen 55' in die poröse Struktur versenkt, wodurch sich zumindest im oberflächennahen Bereich eine durchgehende poröse Struktur ergibt.

Tibiaplatten 91 verschiedener Größe eines vorzugsweise modularen Tibiakomponenten-System sind in Figur 11 dargestellt. Ausgehend von einer kleinsten Größenstufe 9-1 der Tibiakomponente sind sechs verschiedene Größenstufe dargestellt. Bei der kleinsten Größenstufe sind zwei Hauptverstärkungsrippen 55 vorgesehen. Beginnen mit der Größenstufe 9-2 sind bei den mittleren Größenstufe 9-3, 9-4 und 9-5 zur weiteren Aussteifung erste Zusatzrippen 56 vorgesehen. Bei der größten Größenstufe 9-6 sind wegen der zu erwartenden höheren Belastungen zusätzlich noch zweite Zusatzrippen 57 vorgesehen. Die Hauptverstärkungsrippen 55 umschließen jeweils die Befestigungsöffnungen 94, um die durch die dortige Durchbrechung sich ergebende Schwächung der Tibiaplatte 91 zu kompensieren.

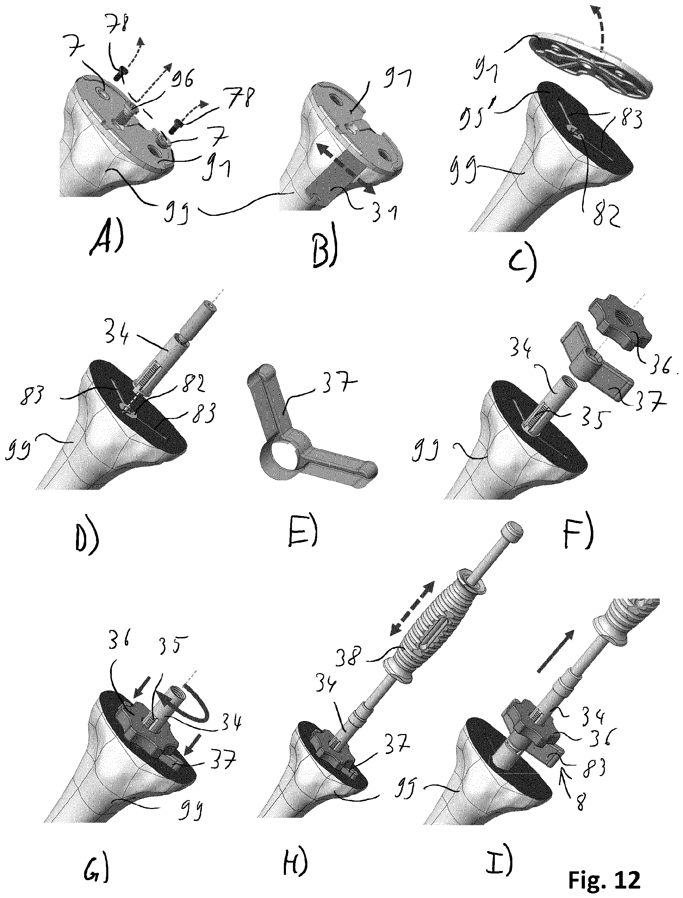

Es wird nun Bezug genommen auf Figur 12, wo Verfahrensschritte zu einer Explantation der Tibiakomponente 9 dargestellt sind. In einem ersten Schritt sind die Befestigungsschrauben 78 sowie die Zentralschraube 96 abzuschrauben und zu entfernen. Dies ist in Figur 12A dargestellt. Im nächsten Schritt werden die Abstandshalter 7 entnommen, wobei in Figur 5 ein Zwischenzustand dargestellt ist, bei dem einer der Abstandshalter 7 entfernt ist und der andere Abstandshalter 7 sich noch in der Tibiaplatte 91 befindet. Die Vollendung dieses Schritts mit entfernten Abstandshaltern 7 ist in Figur 12B dargestellt. Der Freiraum 6 zwischen der unteren, distalen Seite 92 und der Oberkante 85 des Verankerungskiels 8 mit seinen Auslegern 83 ist nun vollständig geöffnet, wie in Figur 6 dargestellt ist (siehe die mit einer gepunkteten Ellipse markierten Bereiche, die den Freiraum 6 ohne die mittlerweile entfernten Abstandshalter 7 zeigen). Dies ist in Figur 12B nicht zu erkennen, da der Freiraum 6 sich innerhalb der Tibia 99 befindet. Zum Freischneiden der Tibiaplatte wird das Sägeblatt 31 einer Säge in den Freiraum 6 bewegt, welcher nun einen Sägeschlitz bildet. Durch Hin- und Herbewegen der Säge wird die Tibiaplatte 91 freigeschnittenen und kann im nächsten Schritt abgenommen werden, wie in Figur 12C dargestellt ist.

Um auch den Verankerungskiel 8 zu entfernen, wird im nächsten Schritt ein stabartiges Kopplungselement 34 auf das Schaftstück 82 aufgeschraubt und zugfest verbunden, wie in Figur 12D dargestellt ist. Im nächsten Schritt wird eine Ausstech-Hülse 37 (siehe Figur 12E), welche als Umhüllende die Außenkontur des Verankerungskiels 8 mit seinen seitlichen Auslegern 83 aufweist, auf das Kopplungselement 34 aufgesetzt. Ferner wird eine Spanneinrichtung, die gebildet ist von einem Außengewinde 35 an dem Kopplungselement 34 und einem Handrad 36 mit Innengewinde angesetzt, wie in Figur 12F dargestellt ist. Durch Drehen des Handrads 36 wird die Spanneinrichtung betätigt und die Ausstech-Hülse 37 in den Tibiakopf getrieben, wie in Figur 12G dargestellt ist. Dabei durchtrennt die Ausstech-Hülse 37 das den Verankerungskiel 8 mit seinen Auslegern 83 haltende Knochengewebe seitlich. Im nächsten Schritt wird ein Gleithammer 38 als Extraktionselement mit dem Koppelelement 34 verbunden, wie in Figur 12H dargestellt ist. Durch Betätigen des Gleithammers 38 kann der durch die Ausstech-Hülse 37 freigeschnittene und gelockerte Verankerungskiel 8 nach proximal ausgetrieben und schließlich herausgezogen werden, wie in Figur 12I dargestellt ist.

## Patentansprüche

1. Tibiakomponente einer Kniegelenkendoprothese umfassend eine Tibiaplatte (91), einen nach distal abragenden Verankerungsschaft (8) und mindestens einem sich nach lateral erstreckenden flügelartigen Ausleger (83),
wobei in einem Implantationszustand die Tibiaplatte (91) und der Verankerungsschaft (8) miteinander verbunden sind, und wobei die Tibiaplatte (91) von dem Verankerungsschaft (8) in einem Explantationszustand abnehmbar ist,
**dadurch gekennzeichnet, dass**
an der Tibiaplatte (91) mindestens ein Abstandshalter (7) vorgesehen ist, wobei eine Stirnseite (72) des Abstandshalters (7) dazu ausgebildet ist, einen Freiraum (6) mit einer definierten Mindestweite zu schaffen.

2. Tibiakomponente nach Anspruch 1, **dadurch gekennzeichnet, dass** eine Befestigungsöffnung (94) eine für den Durchgang des Abstandshalters (7) ausreichende Weite aufweist, wobei vorzugsweise der Abstandshalter (7) im Implantationszustand in eine Befestigungsöffnung (94) der Tibiaplatte (91) eingeschraubt ist, und wobei die Befestigungsöffnung (94) vorzugsweise ausgeführt ist mit graduierter Weite, die sich in proximale Richtung vergrößert.

3. Tibiakomponente nach Anspruch 2, **dadurch gekennzeichnet, dass** der Abstandshalter (7) ein Außengewinde (77) aufweist, das in ein Innengewinde (93) der Befestigungsöffnung (94) eingreift.

4. Tibiakomponente nach Anspruch 2 oder 3, **dadurch gekennzeichnet, dass** der Abstandshalter (7) eine Doppelfunktion hat und auch als Halterung für eine von proximal zu betätigende Befestigungsschraube (78) fungiert, die den Verankerungsschaft (8) mit der Tibiaplatte (91) verbindet.

5. Tibiakomponente nach Anspruch 4, **dadurch gekennzeichnet, dass** die Befestigungsschraube (78) koaxial im Abstandshalter (7) angeordnet ist, wobei sie vorzugsweise in dem Implantationszustand einen Zugang verschließt zu einem Betätigungsorgan (74) des Abstandshalters (7).

6. Tibiakomponente nach einem der vorangehenden Ansprüche, **dadurch gekennzeichnet, dass** dem Ausleger (83) gegenüberliegend ein zweiter Ausleger (83) an dem Verankerungsschaft (8) vorgesehen ist, der an der Tibiaplatte (91) in entsprechender Weise mit einem zweiten Abstandshalter (7) sowie einer zweiten Befestigungsschraube (78) zusammenwirkt, wobei die Ausleger (83) vorzugsweise einen Winkel zwischen 100° und 170° bilden.

7. Tibiakomponente nach einem der vorangehenden Ansprüche, **dadurch gekennzeichnet, dass** der Verankerungsschaft (8) mit den Auslegern (83) eine plane Oberseite (85) aufweist.

8. Tibiakomponente nach einem der vorangehenden Ansprüche, **dadurch gekennzeichnet, dass** an der Tibiaplatte (91) eine Öffnung (97) für eine Zentralschraube (96) vorgesehen ist, mit der der Verankerungsschaft (8) an der Tibiaplatte (91) befestigt ist.

9. Tibiakomponente nach einem der vorangehenden Ansprüche, **dadurch gekennzeichnet, dass** die Tibiaplatte (91) an ihrer distalen Seite (92) mit einer porösen Struktur (5) versehen ist, die vorzugsweise in der Tiefe des Materials miteinander verbundene Poren umfasst, die weiter vorzugsweise eine Weite von 0,1 bis 1,5 mm aufweisen.

10. Tibiakomponente nach Anspruch 9, **dadurch gekennzeichnet, dass** die poröse Struktur (5) vollständig die distale Seite (92) der Tibiaplatte (91) bedeckt.

11. Tibiakomponente nach Anspruch 9, **dadurch gekennzeichnet, dass** hervorstehende Verstärkungsrippen (55) an der distalen Seite (92) der Tibiaplatte (91) vorgesehen sind, die vorzugsweise sich radial von einem Mittelbereich aus zum Rand der Tibiaplatte (91) erstrecken.

12. Tibiakomponente nach Anspruch 9, **dadurch gekennzeichnet, dass** in die poröse Struktur (5) versenkte Verstärkungsrippen (55`) an der distalen Seite (92) der Tibiaplatte (91) vorgesehen sind, die vorzugsweise sich radial von einem Mittelbereich aus zum Rand der Tibiaplatte (91) erstrecken.

13. Tibiakomponente nach Anspruch 9 bis 12, **dadurch gekennzeichnet, dass** an der distalen Seite (92) der Tibiaplatte (91) ein umlaufender Rand (51) für die poröse Struktur (5) vorgesehen ist.

14. Tibiakomponente nach Anspruch 13, **dadurch gekennzeichnet, dass** eine Breite des Rands (51) so bemessen ist, dass die poröse Struktur (5) mindestens die Hälfte, vorzugsweise mehr als 60%, der Oberfläche der distalen Seite (92) der Tibiaplatte (91) einnimmt.

15. Modulares Tibiakomponenten-System von Kniegelenkendoprothesen, umfassend eine oder mehrere Tibiakomponenten (9) nach einem der vorangehenden Ansprüche, **dadurch gekennzeichnet, dass** es mindestens zwei verschiedene Tibiaplatten (91) aufweist, die wahlweise mit einem Verankerungsschaft (8) verbindbar sind, wobei sich die Tibiaplatten (91) hinsichtlich ihrer Ausgestaltung unterscheiden, insbesondere in Bezug auf ihre knochenkontaktierenden Oberflächen.

16. Modulares Tibiakomponenten-System nach Anspruch 15,
**dadurch gekennzeichnet, dass** es die verschiedenen Tibiaplatten (91) in unterschiedlichen Größenstufen umfasst und/oder mindestens zwei verschieden Verankerungsschäfte (8) in unterschiedlichen Größenstufen umfasst.

17. Modulares Tibiakomponenten-System nach Anspruch 16,
**dadurch gekennzeichnet, dass** bei verschiedenen Größenstufen der Tibiaplatten (91) mit poröser Struktur (5) bei kleineren Größenstufen die Breite eines Randes (51) um die poröse Struktur (5) geringer wird an ihrer distalen Seite (92).

18. Instrumentarium für eine Tibiakomponente nach einem der vorangehenden Ansprüche, **dadurch gekennzeichnet, dass** es als Extraktionswerkzeuge umfasst
eine Säge mit einem Sägeblatt (31) oder Sägeband (32), dessen Sägebreite höchstens so groß ist wie die definierte Mindestweite des Freiraums (6), sowie eine Ausstech-Hülse (37), deren Innenkontur zur Aufnahme der lateralen Außenkontur des Verankerungsschafts (8) mit seinen Auslegern (83) ausgebildet ist, und
einem Kopplungselement (34), welches mit dem Schaftstück (82) des Verankerungsschafts (8) zugfest verbindbar ist.
